Europäisches Patentamt

European Patent Office

Office européen des brevets

(11) Publication number: **0 351 698**
**A1**

## EUROPEAN PATENT APPLICATION

(21) Application number: 89112716.9

(22) Date of filing: 12.07.89

(51) Int. Cl.⁴: **A61K 31/71** , //(A61K31/71, 31:44)

(30) Priority: 19.07.88 HU 377288

(43) Date of publication of application:
24.01.90 Bulletin 90/04

(84) Designated Contracting States:
AT BE CH DE ES FR GB GR IT LI LU NL SE

(71) Applicant: CHINOIN Gyogyszer és Vegyészeti Termékek Gyára RT.
To utca 1-5
H-1045 Budapest IV(HU)

(72) Inventor: Kulcsar, Gabor, Dr
138 Szlovak utca
H-1162 Budapest(HU)
Inventor: Sarközy, Péter, Dr
10 Varfok utca
H-1012 Budapest(HU)
Inventor: Zsoldosné, Agnes, Dr
3 Paskompark
H-1157 Budapest(HU)
Inventor: Kaloy, Katalin, Dr
15 Ibrahim utca
H-1113 Budapest(HU)
Inventor: Zilahy, Tibor, Dr
21 Kapy utca
H-1025 Budapest(HU)
Inventor: Schreiner, Enikö, Dr
18 Belgrad rakpart
H-1056 Budapest(HU)

(74) Representative: Lotterhos, Hans Walter, Dr.-Ing.
Lichtensteinstrasse 3
D-6000 Frankfurt am Main 1(DE)

(54) Synergistic pharmaceutical composition with an antimicrobial action and process for preparing same.

(57) The invention relates to a synergistic antimicrobial pharmaceutical composition as well as to a process for preparing this composition. The composition of the invention contains a primycin antibiotic complex and/or a synergistic combination of the isolated components thereof as well as (a) chemotherapeutic agent(s) and optionally (an) other antibiotic(s) in admixture with filling, diluting and formulating materials commonly used in the pharmaceutical industry.

## SYNERGISTIC PHARMACEUTICAL COMPOSITION WITH AN ANTIMICROBIAL ACTION AND PROCESS FOR PREPARING SAME

The invention relates to a synergistic pharmaceutical composition with an antimicrobial action as well as a process for preparing this composition.

The composition according to the invention contains a primycin antibiotic complex, (a) chemotherapeutic agent(s) and optionally (an) other antibiotic(s).

It is known that primycin, an antiobiotic produced by the Thermopolyspora galeriensis actinomycete, forms a synergistic combination with a number of other antibiotics (Hungarian patent specifications Nos. 158,241 and 177,299).

Primycin is an antibiotic complex of several components, i.e. of 3 main or major ones and 10 to 12 minor ones each of which also possesses an antibiotic effect (Hungarian patent specification No. 195,514 and Hungarian patent specification No. 196,425 ). Due to their potentiating effect exerted on the activity of each other, these components are synergistic and thus, a possibility of a combination is given which contains the components in an optimum ratio from the viewpoint of the biological effect (Hungarian patent specification No. 196,309).

The present invention is based on the sur prising recognition that the primycin antibiotic complex or its components, respectively, exert a synergistically potentiating action on a number of chemotherapeutics such as nalidixic acid and/or its derivatives, preferably oxolonic acid, norfloxacin, pefloxacin; furazolidone and/or its derivatives; thiamuline and/or its derivatives; as well as reseptyl and/or its derivatives and the like.

Further on, it has been recognized by the inventors of this application that optionally an other antibiotic, e.g. gentamycin sulfate may also be added to the synergistic composition thus obtained.

Thus, the present invention relates to a synergistic antimicrobial pharmaceutical composition containing as active ingredient a primycin antibiotic complex and/or the isolated components thereof as well as (a) chemotherapeutic agent(s) being synergistic therewith and optionally other antibiotic(s) in admixture with filling, diluting and formulating materials commonly used in the pharmaceutical industry.

Furthermore, the invention relates to a process for the preparation of these compositions which comprises mixing the active ingredients with filling, diluting and formulating materials commonly used in the pharmaceutical industry.

A double combination according to the invention contains 5 to 50 % of primycin antibiotic complex and/or a synergistic combination of its isolated components as well as 50 to 95 % of (a) chemotherapeutic agent(s) as active ingredients. A triple combination according to the invention contains 2 to 35 % of primycin antibiotic complex and/or a synergistic combination of its isolated components as well as 30 to 60 % of (a) chemotherapeutic agent(s) and 20 to 65 % of (an) other antibiotic(s) as active ingredients.

In the composition according to the invention, as chemotherapeutic agent(s) nalidixic acid and/or its derivatives, preferably oxolinic acid, norfloxacin, perfloxacin or furazolidone and/or its derivatives or thiamuline and/or its derivatives or reseptyl and/or its derivatives and as antibiotic, gentamycin sulfate are used.

For the formulation of the active ingredients, carriers such as magnesium carbonate, magnesium stearate, starch, talc and the like or cyclodextrin as a novel carrier and optionally other auxiliary material(s), e.g. filling, disintegrating, lubricating and emulsifying agents commonly used in the formulation of pharmaceutical compositions may also be employed.

The pharmaceutical compositions according to the invention can be formulated as solid (such as tablet, capsule, suppository and the like), semisolid (e.g. ointment) or liquid (such as an injectable solution, suspension, emulsion) preparations. It is pre ferred to formulate the compositions according to the invention to a gel, ointment, wound-dusting powder, injectable solution, suspension or as a combination of a powder ampoule with a solvent ampoule.

The advantages of the synergistic pharmaceutical compositions according to the invention can be summarized as follows:

1) The pathogenes are simultaneously attached by the active ingredients of the composition on several sites of their metabolism; thereby, a "cidal" effect is ensured which means the perishment of the pathogens in opposition to the "static" effect involving only the inhibition of their development.

2) The simultaneous attach on several sites of metabolism of the pathogens delays to a great extent the development of any resistance to the combination.

3) Due to the synergistic action occurring between the active ingredients, the amount required to fight the individual pathogens, i.e. the minimum inhibitory concentration (MIC value) of the composition becomes a significantly lower. Thus, substantially lower amounts of the separate active ingredients have to be introduced whereby the individual harmful side effects are diminished or even eliminated.

4) The spectrum of effects is significantly broadened by using the synergistic compositions.

5) Due to the necessity of lower amounts of the active ingredients their use is economic and environment-sparing.

The synergistic pharmaceutical compositions according to the invention can be administered in an oral, parenteral or rectal route or topically (e.g. in the form of an ointment, spray, dusting powder, emulsion, suspension and the like).

The pharmaceutical compositions containing the synergistic combination of the active ingredients can be used in the veterinary medicine, too, e.g. in the form of a powder mixture mixed to the fodder or as a solution mixed to the drinking liquid or in a parenterial route, e.g. in the form of solutions, aqueous emulsions and suspensions; or topically, in the form of an ointment, aqueous or oily suspensions or emulsions, spray, dusting powder and the like.

The biological activity of the pharmaceutical compositions according to the invention was studied in vitro on international (internationally accepted) resistant and/or polyresistant human and/or animal pathogens. (The strains investigated are listed in Table I.) These investigations were carried out by using: a Difco Bouillon medium for bacteria; a modi fied Difco Bouillon medium for vibrios; and Sabouraud's medium for fungi. The inoculation was carried out by a germ number of $5 \times 10^5$ cell/ml. The inoculation lasted 24 hours at 37 °C.

## Table I

The strains investigated were signed and numbered
as follows:

|     |                                      |              |
| --- | ------------------------------------ | ------------ |
| 1.  | Staphylococcus aureus                | CCM 2514     |
| 2.  | Staphylococcus aureus                | CCM 2317     |
| 3.  | Staphylococcus aureus                | CCM  885     |
| 4.  | Streptococcus disgalactiae           | CCM 5548     |
| 5.  | Streptococcus agalactiae             | CCM 5153     |
| 6.  | Streptococcus agalactiae             | CCM 5534     |
| 7.  | Streptococcus faecalis               | CCM 1875     |
| 8.  | Bacillus cereus                      | CCM 2010     |
| 9.  | Bacillus licheniformis               | CCM 2182     |
| 10. | Bacillus licheniformis               | CCM 2205     |
| 11. | Micrococcus flavus                   | ATCC 10240   |
| 12. | Pseudomonas aeruginosa               | CCM 1960     |
| 13. | Pseudomonas fluorescens              | CCM 2115     |
| 14. | Pseudomonas acidovorus               | CCM  283     |
| 15. | Pseudomonas pictorum                 | CCM  284     |
| 16. | Pseudomonas fluorescens-putida       | M-III-21     |
| 17. | Pseudomonas putrefaciens             | Sz-III-156   |

4

| 18. Proteus vulgaris | CCM 1799 |
| 19. Escherichia coli | CCM 5863 |
| 20. Escherichia coli | CCM 5172 |
| 21. Escherichia coli | CCM 186 |
| 22. Escherichia coli | DSM 30038 |
| 23. Escherichia coli | OTKI 17963 |
| 24. Escherichia coli | OTKI 22143 |
| 25. Escherichia coli | OTKI 23473 |
| 26. Klebsiella pneumoniae | CCM 1848 |
| 27. Shigella sonnei | CCM 1373 |
| 28. Salmonella cholerae-suis | CCM 5438 |
| 29. Serratia marcescens | CCM 303 |
| 30. Pasteurella multocida | CCM 5419 |
| 31. Vibrio parahaemoliticus | CCM 5938 |
| 32. Vibrio mariganilis | ATCC 14398 |
| 33. Vibrio fischeri | ATCC 15382 |
| 34. Vibrio haloplanctis | ATCC 14393 |
| 35. Vibrio cuneatus | ATCC 6972 |
| 36. Vibrio albensis | ATCC 14547 |
| 37. Vibrio algosus | ATCC 14390 |
| 38. Vibrio marinofulvus | ATCC 14395 |
| 39. Vibrio alginolyticus | CCM 2578 |
| 40. Candida albicans | CBS 562 |
| 41. Candida tropicalis | CBS 433 |
| 42. Candida pseudotropicalis | |
| 43. Cryptococcus neoformans | 78/K.16 |

Within the study of the biological activity, the minimum concentration value required to achieve an inhibitory effect against the individual strains of pathogenic microorganisms (i.e. the MIC value) was determined in $\mu$g/ml term.

First, the separately measured MIC value of each active ingredient (a, b and c, respectively) of the pharmaceutical compositions according to the invention was determined. Then, the amount of each active ingredient was determined which, when used in a combination (A + B, or + C, respectively) produced the same inhibitory effect as used in itself against the microorganism given.

The percentage ratio of the MIC value measured in combination in relation to the MIC value measured with the separate active ingredients was calculated in the case of each active ingredient. The results of the biological study are summarized in Tables II to VII.

Table II

The biological activity of primycin + oxolinic acid on the basis of the MIC values (µg/ml) measured on human and animal pathogenic microorganism strains

| No. of strains investigated | MIC value (µg/ml) | | Combination | | % ratio of active ingredients | |
|---|---|---|---|---|---|---|
| | Pr. a | Ox. b | Pr. A | Ox. B | A/a | B/b |
| 3 | 0.25 | 25 | 0.075 | 10 | 30 | 40 |
| 4 | 5 | 25 | 0.25 | 2.5 | 5 | 10 |
| 5 | 2.5 | 100 | 0.5 | 10 | 20 | 10 |
| 12 | 25 | 150 | 10 | 10 | 40 | 6.6 |
| 13 | 25 | 10 | 1 | 2.5 | 4 | 25 |
| 14 | 25 | 0.25 | 5 | 0.05 | 20 | 20 |
| 18 | 25 | 1 | 5 | 0.5 | 20 | 50 |
| 19 | 50 | 5 | 25 | 1 | 50 | 20 |
| 20 | 50 | 2.5 | 10 | 1 | 20 | 40 |
| 21 | 50 | 5 | 10 | 0.5 | 20 | 10 |
| 23 | 25 | 5 | 10 | 0.5 | 40 | 10 |
| 26 | 50 | 5 | 5 | 0.5 | 10 | 10 |

6

Table II. (contd.)

| No. of strains investigated | MIC value (µg/ml) | | | | % ratio of active ingredients | |
|---|---|---|---|---|---|---|
| | Pr. | Ox. | Combination Pr. + Ox. | | A/a | B/b |
| | a | b | A + | B | | |
| 29 | 25 | 2.5 | 0.5 | 0.5 | 2 | 20 |
| 30. | 25 | 2.5 | 10 | 0.1 | 40 | 4 |
| 40. | 25 | 200 | 10 | 50 | 40 | 25 |
| 41. | 25 | 150. | 10 | 10 | 40 | 6.6 |
| 42. | 5 | 50 | 1 | 5 | 20 | 10 |
| 43. | 5 | 75 | 1 | 25 | 20 | 3.33 |
| 32 | 1 | 0.5 | 0.05 | 0.05 | 5 | 10 |
| 33 | 1 | 0.5 | 0.05 | 0.05 | 5 | 10 |
| 34 | 5 | 0.5 | 0.125 | 0.125 | 2.5 | 25 |
| 35 | 10 | 25 | 5 | 5 | 50 | 20 |
| 36 | 25 | 0.25 | 0.125 | 0.125 | 0.5 | 50 |
| 37 | 2.5 | 0.5 | 0.05 | 0.05 | 2 | 10 |
| 38 | 1 | 0.25 | 0.05 | 0.05 | 5 | 20 |
| 39 | 50 | 2.5 | 1.25 | 1.25 | 2.5 | 50 |

Abbreviations: Pr: primycin

Ox: oxolinic acid

## Table III

The biological activity of primycin + norfloxacin on the basis of the MIC values ($\mu$g/ml) measured on human and animal pathogenic microorganism strains

| No. of strains investigated | MIC value ($\mu$g/ml) | | Combination | | | % ratio of active ingredients | |
|---|---|---|---|---|---|---|---|
| | Pr. a | Norf. b | Pr. A | + | + Norf. B | A/a | B/b |
| 22 | 50 | 0.25 | 5 | | 0.075 | 10 | 30 |
| 26 | 50 | 0.1.25 | 5 | | 0.1 | 10 | 40 |
| 18 | 25 | 0.5 | 7.5 | | 0.1 | 30 | 20 |
| 17 | 10 | 0.75 | 2.5 | | 0.1 | 25 | 13.3 |
| 16 | 50 | 0.5 | 10 | | 0.25 | 20 | 50 |
| 30 | 25 | 0.5 | 7.5 | | 0.1 | 30 | 20 |
| 2 | 0.1 | 5 | 0.01 | | 0.75 | 10 | 15 |
| 4 | 5 | 10 | 0.75 | | 0.75 | 15 | 7.5 |
| 8 | 0.1 | 1 | 0.01 | | 0.5 | 10 | 50 |

## Table III

### (contd.)

| No. of strains investigated | MIC value (µg/ml) | | | | % ratio of active ingredients | |
|---|---|---|---|---|---|---|
| | | | Combination | | A/a | B/b |
| | Pr. | Norf. | Pr. + | Norf. | | |
| | a | b | A + | B | | |
| 9 | 0.075 | 0.75 | 0.0075 | 0.25 | 10 | 33.3 |
| 10 | 0.075 | 0.25 | 0.025 | 0.075 | 33.3 | 30 |

Abbreviations: Pr: primycin

Norf: norfloxacin

EP 0 351 698 A1

## Table IV

The biological activity of primycin + furazolidone on the basis of the MIC values ($\mu$g/ml) measured on human and animal pathogenic microorganism strains

| No. of strains investigated | MIC value ($\mu$g/ml) | | | | % ratio of active ingredients | |
|---|---|---|---|---|---|---|
| | Pr. | Fur. | Combination | | A/a | B/b |
| | a | b | Pr. + Fur. A + B | | | |
| 31 | 25 | 2.5 | 2.5 | 0.25 | 10 | 10 |
| 13 | 25 | 150 | 5 | 10 | 20 | 6.6 |
| 14 | 25 | 25 | 5 | 5 | 20 | 20 |
| 15 | 10 | 25 | 2.5 | 10 | 25 | 40 |
| 18 | 25 | 25 | 7.5 | 7.5 | 30 | 30 |
| 27 | 25 | 2.5 | 2.5 | 1 | 10 | 40 |
| 28 | 50 | 5 | 7.5 | 2.5 | 15 | 50 |
| 19 | 50 | 25 | 10 | 5 | 20 | 20 |
| 20 | 50 | 2.5 | 10 | 1 | 20 | 40 |
| 21 | 50 | 2.5 | 10 | 1 | 20 | 40 |

## Table IV

### (contd.)

| No. of strains investigated | MIC value ($\mu$g/ml) | | | | % ratio of active ingredients | |
|---|---|---|---|---|---|---|
| | | | Combination | | A/a | B/b |
| | Pr. | Fur. | Pr. + | Fur. | | |
| | a | b | A + | B | | |
| 24 | 50 | 2.5 | 10 | 1 | 20 | 40 |
| 23 | 25 | 5 | 10 | 1 | 40 | 20 |
| 25 | 50 | 5 | 10 | 1 | 20 | 20 |
| 26 | 50 | 2.5 | 10 | 1 | 20 | 40 |
| 40 | 25 | 100 | 10 | 25 | 40 | 25 |
| 42 | 5 | 25 | 1 | 10 | 20 | 40 |
| 43 | 5 | 75 | 2.5 | 10 | 50 | 13.3 |

Abbreviations: Pr: primycin

Fur: furazolidone

EP 0 351 698 A1

EP 0 351 698 A1

## Table V

The biological activity of primycin + reseptyl on the basis of the MIC values ($\mu$g/ml)

measured on human and animal pathogenic microorganism strains

| No. of strains investigated | MIC value ($\mu$g/ml) | | | | % ratio of active ingredients | |
|---|---|---|---|---|---|---|
| | Pr. a | Res. b | Combination Pr. A + | Res. + B | A/a | B/b |
| 31 | 25 | 150 | 2.5 | 10 | 10 | 6.6 |
| 12 | 25 | 200 | 10 | 50 | 40 | 25 |
| 13 | 25 | 150 | 5 | 25 | 20 | 16.6 |
| 14 | 25 | 150 | 5 | 25 | 20 | 16.6 |
| 18 | 25 | 200 | 5 | 50 | 20 | 25 |
| 2C | 50 | 200 | 10 | 50 | 20 | 25 |
| 24 | 50 | 200 | 10 | 75 | 20 | 37.5 |
| 25 | 50 | 200 | 10 | 75 | 20 | 37.5 |
| 30 | 25 | 25 | 2.5 | 5 | 10 | 20 |

Table V

(contd.)

| No. of strains investigated | MIC value ($\mu$g/ml) | | | | | % ratio of active ingredients | |
|---|---|---|---|---|---|---|---|
| | | | Combination | | | A/a | B/b |
| | Pr. | Res. | Pr. | + | Res. | | |
| | a | b | A | + | B | | |
| 4 | 5 | 150 | 1 | | 25 | 20 | 16.6 |
| 5 | 2.5 | 150 | 1 | | 10 | 40 | 6.6 |
| 42 | 5 | 50 | 2.5 | | 10 | 50 | 20 |
| 43 | 5 | 75 | 2.5 | | 10 | 50 | 13.3 |

Abbreviations: Pr: primycin

Res: reseptyl

...

## Table VI

The biological activity of primycin + thiamuline on the basis of the MIC values ($\mu$g/ml) measured on human and animal pathogenic microorganism strains

| No. of strains investigated | MIC value ($\mu$g/ml) | | | | % ratio of active ingredients | |
| --- | --- | --- | --- | --- | --- | --- |
| | | | Combination | | | |
| | Pr. | Thia. | Pr. + | Thia. | A/a | B/b |
| | a | b | A + | B | | |
| 31 | 25 | 100 | 10 | 25 | 40 | 25 |
| 13 | 25 | 50 | 10 | 7.5 | 40 | 15 |
| 14 | 25 | 25 | 2.5 | 2.5 | 10 | 10 |
| 15 | 10 | 5 | 1 | 0.5 | 10 | 10 |
| 18 | 25 | 100 | 10 | 25 | 40 | 25 |
| 19 | 50 | 50 | 10 | 25 | 20 | 50 |
| 23 | 25 | 50 | 5 | 25 | 20 | 50 |
| 25 | 50 | 50 | 10 | 25 | 20 | 50 |
| 2 | 0.1 | 1 | 0.01 | 0.25 | 10 | 25 |

EP 0 351 698 A1

## Table VI

### (contd.)

| No. of strains investigated | MIC value (μg/ml) | | | | % ratio of active ingredients | |
|---|---|---|---|---|---|---|
| | Combination | | | | A/a | B/b |
| | Pr. a | Thia. b | Pr. A | + | Thia. + B | |
| 1 | 0.25 | 0.1 | 0.075 | | 0.01 | 30 | 10 |
| 7 | 2.5 | 10 | 1 | | 1 | 40 | 10 |
| 6 | 2.5 | 0.25 | 0.5 | | 0.025 | 20 | 10 |
| 4 | 5 | 0.1 | 0.5 | | 0.01 | 10 | 10 |
| 5 | 2.5 | 1 | 0.75 | | 0.1 | 30 | 10 |
| 8 | 0.1 | 75 | 0.05 | | 10 | 50 | 13.3 |
| 11 | 0.1 | 10 | 0.01 | | 1 | 10 | 10 |

Abbreviations: Pr: primycin

Thia: thiamuline

Table VII

| The biological activity of primycin + oxolinic acid + gentamycin on the basis of the MIC vales (μg/ml) measured on human and animal pathogenic microorganism strains | | | | | | | | | |
|---|---|---|---|---|---|---|---|---|---|
| No. of strains investigated | MIC value (μg/ml) | | | | | | % ratio of active ingredients | | |
| | | | | Combination | | | | | |
| | Pr | Ox. | Gent. | Pr + | Ox + | Gent | | | |
| | a | b | c | A + | B + | C | A/a | B/b | C/c |
| 31 | 25 | 1 | 10 | 1 | 0.1 | 1 | 4 | 10 | 10 |
| 12 | 25 | 150 | 0.75 | 2.5 | 10 | 0.075 | 10 | 6.6 | 10 |
| 13 | 25 | 10 | 0.75 | 2.5 | 1 | 0.075 | 10 | 10 | 10 |
| 20 | 50 | 2.5 | 0.75 | 5 | 0.25 | 0.075 | 10 | 10 | 10 |
| 26 | 50 | 5 | 0.25 | 5 | 0.75 | 0.075 | 10 | 15 | 30 |
| 29 | 25 | 2.5 | 1 | 5 | 0.5 | 0.1 | 20 | 20 | 10 |
| 30 | 25 | 2.5 | 1 | 5 | 0.1 | 0.25 | 20 | 4 | 25 |
| 4 | 5 | 25 | 0.75 | 0.75 | 1 | 0.1 | 20 | 4 | 13.3 |
| 9 | 0.075 | 2.5 | 0.25 | 0.0075 | 0.5 | 0.025 | 10 | 20 | 10 |
| Abbreviations: Pr: primycin   Ox: oxolinic acid   Gent: gentamycin | | | | | | | | | |

The invention is illustrated in detail by the following non-limiting Examples.

| Example 1 | |
|---|---|
| **Preparation of a granulate** | |
| | g |
| Primycin sulfate | 166.5 |
| Gentamycin sulfate | 166.5 |
| Nalidixic acid | 499.5 |
| White gelatine | 300.0 |
| Lactose | 6213.5 |
| Maize starch | 2654.0 |
| | 10000.0 |
| Distilled water as required, about | 2000 to 3000 |

The preparation is carried out as follows.

White gelatine is swollen in the distilled water and dissolved on a hot water bath. The water evaporating during the dissolution and stirring is supplemented. For granulation, a gelatine mucus of 40 °C is used (I).

The active ingredients are weighed and homogenized (II).

The filling materials are weighed, homogenized and, if desired, sieved (III).

The active ingredients (II) and filling materials (III) homogenized are filled into e.g. an apparatus equipped with a stirrer of "Z"-shaped arm and kneaded together with the gelatine mucus (I) of about 40 °C.

| Example 2 | |
|---|---|
| Preparation of a granulate | |
| | g |
| Oxolinic acid | 100.0 |
| Gentamycin sulfate | 166.0 |
| Heterocolloidal primycin (containing primycin sulfate) | 16.0 |
| White gelatine | 300.0 |
| Lactose | 2380.0 |
| Maize starch | 7038.0 |
| | 10000.0 |
| Distilled water as required, about | 200.0 |

The preparation is carried out as follows.

The maize starch lactose and gentamycin sulfate are filled into a kneader, the heterocolloidal primycin solution (prepared as described in the Hungarian patent specification No. 173,708, corresponds to the GB patent specification No. 1.512.604) and oxolinic acid are mixed thereto and homogenized. Then, the mixture is granulated together with the gelatine mucus prepared as described in Example 1, dried and re-granulated. The product obtained divided to 3 g portions each is filled into bags. The granulate obtained can be mixed into the fodder and/or suspended in milk.

| Example 3 | |
|---|---|
| Preparation of an ointment | |
| | g |
| Primycin sulfate, micronized | 2.0 |
| Oxolinic acid | 10.0 |
| Gentamycin sulfate | 20.0 |
| White wax | 50.0 |
| White vaseline | 338.0 |
| Lanolin | 290.0 |
| Liqiud paraffin | 290.0 |
| | 1000.0 |

The active ingredients are homogenized, sieved and then triturated with about 50 to 100 g of liquid paraffin (I).

The white wax, white vaseline, lanolin and the remained liquid paraffin are melted together, filtered if necessary and then stirred until it is cooled down (II).

The active ingredients triturated with liquid paraffin (I) are mixed together with the carriers (II).

The mixture obtained is homogenized in a three-roller-mill then filled in suitable tubes and sealed.

| Example 4 | |
|---|---|
| Preparation of an ointment | |
| | g |
| Gentamycin sulfate | 20.0 |
| Tolnaphthate | 20.0 |
| Heterocolloidal primycin (containing 1.0 of primycin sulfate) | 100.0 |
| Nonionic hydrophilic ointment according to the Hungarian Pharmacopoea VI. | up to 1000.0 |

The preparation is carried out as follows.

17

Gentamycin sulfate and tolnaphthate are homogenized and sieved. The powder thus prepared is triturated with little portions of the ointment and heterocolloidal primycin solution. If necessary, the ointment is homogenized in a three-roller-mill, then filled in suitable tubes and sealed.

| Example 5 | |
|---|---|
| Preparation of a spray | |
| | g |
| Primycin sulfate | 1.0 |
| Gentamycin sulfate | 1.0 |
| Nalidixic acid | 1.0 |
| Isopropyl myristate | 5.0 |
| Freon $^R$ 11/12 5050 | up to 100.0 |

The preparation is carried out as follows.

The active ingredients are dried and then pretreated in such a way that the linear size of the granules do not exceed 10 µm. The active ingredients are triturated with isopropyl myristate and then filled into aluminum bottles lacquered inside. The bottles are sealed with suitable nozzles and the appropriate driving gas (in this case Freon 11/12 5050) is filled onto.

| Example 6 | |
|---|---|
| Preparation of a paste | |
| | g |
| Primycin sulfate | 0.5 |
| Gentamycin sulfate | 0.5 |
| Nalidixic acid | 1.5 |
| Carboxymethylcellulose sodium | 3.0 |
| Saccharose | 5.0 |
| Stabilizing solution | 1.0 |
| Flavouring agent | as required |
| Distilled water | up to 100.0 |

The preparation is carried out as follows.

Carboxymethylcellulose sodium is mixed with 20 g of hot distilled water and after cooling down, it is maintained in a refrigerator at -5 °C for 24 hours. After thawing of the gel, the suspended and water-soaked active ingredients are mixed thereto, the flavouring and stabilizing solutions are added, homogenized and filled up to 100 g by adding distilled water.

The paste thus obtained is filled into a suitable plastic feeding syringe and administered in portions of 1 g each to e.g. pigs.

## Claims

1. Synergistic antimicrobial pharmaceutical composition, which comprises as active ingredient a primycin antibiotic complex and/or a synergistic combination of the isolated components thereof as well as (a) chemotherapeutic agent(s) and optionally (an) other antibiotic(s) in admixture with filling, diluting and formulating materials commonly used in the pharmaceutical industry.

2. A pharmaceutical composition as claimed in claim 1 in the form of a double combination, which comprises as active ingredient 5 to 50 % of primycin antibiotic complex and/or a synergistic combination of the isolated components thereof together with 95 to 50 % of (a) chemotherapeutic agent(s).

3. A pharmaceutical composition as claimed in claim 1 in the form of a triple combination, which comprises as active ingredient 2 to 35 % of primycin antibiotic complex and/or a synergistic combination of the isolated components thereof together with 30 to 60 % of (a) chemotherapeutic agent(s) and 20 to 65 % of (an) other antibiotic(s).

4. A pharmaceutical composition as claimed in any of the claims 1 to 3, which comprises as chemotherapeutically active ingredient nalidixic acid and/or derivatives thereof, preferably oxolinic acid, norfloxacin, perfloxacin or furazolidone and/or derivatives thereof or thiamuline and/or derivatives thereof of reseptyl and/or derivatives thereof.

5. A pharmaceutical composition as claimed in any of claims 1 to 3 in the form of a triple combination, which comprises gentamycin sulfate as an other antibiotic.

6. A pharmaceutical composition as claimed in any of the claims 1 to 5 in the form of an ointment, paste, spray, dusting powder or other topically useful pharmaceutical preparation, or in the form of granules, or injectable preparations, or in the form of a combination of a powder ampoule and solvent ampoule, or in the form of a tablet, capsule or suppository.

7. A process for the preparation of a synergistic antimicrobial pharmaceutical composition, which comprises mixing as active ingredients a primycin antibiotic complex and/or a synergistic combination of the isolated components thereof as well as (a) chemotherapeutic agent(s) and optionally (an) other antibiotic(s) with filling, diluting and formulating materials commonly used in the pharmaceutical industry.

8. A process as claimed in claim 7, which comprises formulating 1 to 10 % amount of active ingredient consisting of primycin antibiotic complex and/or a synergistic combination of the isolated com ponents thereof as well as (a) chemotherapeutic agent(s) and optionally (an) other antibiotic(s) with 90 to 99 % amount of filling, diluting and formulating auxiliary materials commonly used in the pharmaceutical industry.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 104 282  (C. GYOGYSZER) <br> * Page 1, claim 1; page 26, example * <br> --- | 1-8 | A 61 K  31/71 // <br> (A 61 K  31/71 <br> A 61 K  31:44 ) |
| Y | EP-A-0 224 868  (C. GYOGYSZER) <br> * Page 13, lines 35-43; claims 9-10 * <br> --- | 1-4 | |
| Y | FR-A-2 439 015  (AUSONIA FARMACEUTICI S.R.L.) <br> * Pages 1-2 * <br> --- | 1-4 | |
| A | GB-A-2 103 085  (C. GYOGYSZER) <br> ----- | 1-8 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 20-10-1989 | BRINKMANN C. |